# EUROPEAN PATENT SPECIFICATION

(11) **EP 0 297 769 B1**
(45) Date of publication and mention of the grant of the patent: **17.08.1994**
(21) Application number: 88305655.8
(22) Date of filing: 20.06.1988
(51) Int. Cl.: A61L 15/58, A61L 15/60, A61L 25/00

(54) **Process for preparing a wound dressing comprising a hydrophilic acrylic adhesive layer.**
Verfahren zur Herstellung eines Pflasters mit einer hydrophilen Acrylat-Klebstoff Schicht.
Procédé de fabrication d'un pansement comprenant une couche adhésive acrylique hydrophile.

(30) Priority: 29.06.1987 US 67328
(43) Date of publication of application: 04.01.1989
(73) Proprietor: E.R. Squibb & Sons, Inc., Princeton, N.J. 08540-4000 (US)
(72) Inventor: Cilento, Rodolfo Dominic, North Brunswick New Jersey (US)
(74) Representative: Thomas, Roger Tamlyn

(56) References cited:
- EP-A- 0 035 399
- EP-A- 0 122 344
- EP-A- 0 175 562
- EP-A- 0 275 550
- EP-A- 0 279 118
- US-A- 4 307 717
- US-A- 4 505 976

## Description

This invention provides a process for preparing a wound dressing comprising a hydrophilic adhesive layer suitable for use on the human skin comprising a pressure sensitive acrylic adhesive mass containing one or more water/moisture absorbing, water/moisture transmitting substances, wherein said water/moisture absorbing, water/moisture transmitting substances are (i) one or more hydrocolloids selected from sodium carboxymethylcellulose, calcium carboxymethylcellulose, pectin, gelatin, guar gum, locust bean gum, collagen, and gum karaya, (ii) one or more super absorbents selected from substantially water insoluble starch-acrylonitrile graft copolymers, substantially water insoluble cross-linked sodium carboxymethylcellulose, and substantially water insoluble cross-linked dextran, or (iii) mixtures of one or more of said hydrocolloids and super absorbents, said process comprising:
a) adding a solution of the pressure sensitive acrylic adhesive in an organic solvent to a propeller type mixer,
b) blending into said acrylic solution a powder comprising the one or more water/moisture absorbing, water/moisture transmitting substances,
c) continuing said blending until a homogeneous slurry is formed,
d) casting the resulting slurry of hydrophilic, pressure sensitive, adhesive onto a sheet of release carrier, to a thickness which provides from 40 to 100 mg of hydrophilic, pressure sensitive, adhesive per square inch (6.452 x 10⁻⁴ m²) after drying,
e) drying said adhesive slurry to evaporate off said organic solvent, and
f) laminating the resulting hydrophilic adhesive layer to a polymeric foam layer.

Pressure sensitive adhesive compositions particularly suited for adherence to moist body surface have been disclosed. For example, United States Patent US-A-3,339,546 discloses such a composition prepared by blending a natural or synthetic viscous gum like substance such as natural rubber, silicone rubber, acrylonitrile rubber, polyisobutylene, which is preferred, etc., and one or more water soluble or swellable hydrocolloids such as sodium carboxymethylcellulose, pectin, gelatin, etc.

United States Patent US-A-4,192,785 discloses a pressure sensitive adhesive composition particularly useful for attaching an ostomy appliance to the skin. The composition is a blend of certain moisture absorbing hydrocolloid gums such as guar gum or locust bean gum, a pressure sensitive adhesive component such as low molecular weight polyisobutylene or mixtures of low molecular weight polyisobutylene and butyl rubber or medium molecular weight polyisobutylenes, a cohesive strengthening agent such as fibrous materials, cellulosic materials, water insoluble cross-linked dextran, water insoluble cross-linked sodium carboxymethylcellulose, or water-insoluble starch-acrylonitrile graft copolymers, and optionally one or more skin soothing or healing hydrocolloid gums such as pectin or karaya.

United States Patent US-A-4,551,490 discloses a pressure sensitive adhesive composition particularly resistant to biological fluids. The composition is a blend of one or more polyisobutylenes or polyisobutylene and butyl rubber, one or more styrene radial or block copolymers, mineral oil, one or more water soluble hydrocolloid gums, and a tackifier.

United States Patent US-A-3,972,328 and United States Patent US-A-4,538,603 disclose dressings or bandages having a skin and wound contacting pressure sensitive adhesive layer and a layer of semi-open cell polyurethane foam. The skin contacting adhesive layer contains one or more hydrocolloids dispersed in a rubbery elastomer such as polyisobutylene.

European Patent Application EP-A-190,814 disclose a wound dressing comprising a closed cell polyurethane foam containing from about 5% to about 50% by weight of the foam of one or more water dispersible, water swellable, and/or water absorbing agents. A pressure sensitive adhesive is laminated as a continuous or discontinuous layer onto one surface of the foam. The adhesive can be an acrylic pressure sensitive adhesive, an acrylic microporous pressure sensitive adhesive, or a polyisobutylene-hydrocolloid containing pressure sensitive adhesive.

Pressure sensitive adhesives have been formulated from acrylics. For example, United States Patent RE24,906 discloses an acrylic based pressure sensitive adhesive from a copolymer of isooctyl acrylate and acrylic acid. Various pressure sensitive acrylic adhesives suitable for use in bandages and wound dressing are disclosed in United States Patents RE31,886 and 31,887, and in United States Patents US-A-3,475,363, 3,928,262, 3,975,570, 3,983,297, 4,379,881 and 4,510,197.

United States Patent US-A-4,307,717 discloses an adhesive bandage containing a medicament. The bandage comprises a flexible backing and a hydrophilic adhesive matrix comprising about 30 to 50% of polyacrylic acid, polyacrylamide and their cogeners and a liquid phase consisting of a solution or emulsion of carbohydrate and/or protein, and a medicament such as an anti-inflammatory agent. The solid phase of the matrix can include a gum such as karagen, gum acacia, locust bean gum, and guar gum.

United States Patent US-A-4,505,976 discloses a pressure-sensitive adhesive, particularly for use as a stoma seal adhesive, which has good wet-stick and which includes a gum-like substance, a moisture absorbing component and silica. Acrylic polymers are given as one example of the gum-like substance.

European Patent Application EP-A-122344 discloses a medical grade adhesive composition comprising a homogeneous blend of one or more pressure sensitive adhesive materials and one or more natural or synthetic polymers capable of developing elastomeric properties when hydrated such as gluten and long chain polymers of methyl vinyl ether/maleic acid are disclosed. The composition may also include one or more water soluble hydrocolloid gums and may additionally contain one or more water swellable cohesive strengthening agents. The composition is initially prepared as a dough-like mass which is then extruded and rolled or pressed.

European Patent Application EP-A-279118 which was published after the filing date of the present application and is cited under article 54(3)EPC, discloses an adhesive product suitable for use on wounds which comprises a backing layer of a moisture vapour transmitting polymer film and a layer of pressure adhesive containing at least 30% by weight of acrylic adhesive and at least 30% by weight of alginate.

European Patent Application EP-A-275550 which was also published after the filing date of the present application (Art 54(3)EPC), discloses an adhesive device for application to body tissue having an adhesive layer and a backing layer positioned over one side of the adhesive layer. The adhesive layer includes one or more acrylic acid polymers having adhesive properties upon dissolution or swelling in water, and at least one water insoluble cellulose derivative. The backing layer is water insoluble or sparingly water soluble.

Acrylic adhesive formulations possess several properties that make them particularly useful in medical applications. Acrylic adhesive can be irradiated for sterilization purposes without any significant change in their adhesive properties. Acrylic adhesives are temperature stable and are less prone to cold flow, i.e., the creeping of the adhesive mass away from a backing film, than polylsobutylene based adhesives.

However, despite these advantages, acrylic adhesives suffer from a serious drawback in their lack of adhesion to moist body surfaces and their inability to retain adhesive strength in the presence of moistness. Thus, perspiration which can form under or along the edges of the adhesive layer, moisture from wound exudate, or external moisture from showering or bathing can result in a loss of adhesive strength.

This invention employs pressure sensitive hydrophilic acrylic adhesives suitable for use on human skin which retain their adhesive strength in the presence of moisture and/or wound exudate. This result is achieved by incorporating one or more water/moisture absorbing, water/moisture transmitting substances within the acrylic adhesive mass.

Hydrophilic, pressure sensitive, acrylic adhesives suitable for various medical applications are prepared by blending one or more water/moisture absorbing, water/moisture transmitting substances into the acrylic adhesive mass. Suitable water/moisture absorbing, water/moisture transmitting substances include water soluble and/or water swellable hydrocolloids, one or more "super absorbents", or a mixture of hydrocolloids and "super absorbents".

Suitable hydrocolloids include sodium carboxymethylcellulose, calcium carboxymethylcellulose, pectin, gelatin, guar gum, locust bean gum, collagen, and gum karaya. These substances result in the acrylic adhesive becoming hydrophilic when present at from 15% to 50% by weight of the acrylic adhesive compositions, preferably from 20% to 40% by weight of the adhesive composition.

The term "super absorbent" refers to agents capable of absorbing water/moisture in amounts greater than their own weight. Suitable "super absorbents" include substantially water insoluble starch-acrylonitrile graft copolymers such as those described in United States Patent US-A-3,661,815 and those available commercially under the trademark Water Lock from the Grain Processing Corp., water insoluble cross-linked sodium carboxymethylcellulose such as that commercially available under the trademark Aqualon or that described in United States Patent US-A-3,589,364 and commercially available from the Buckeye Cellulose Corp., and substantially water insoluble cross-linked dextran such as that commercially available under the trademark Sephadex. The acrylic adhesive becomes hydrophilic upon the addition of from 5% to 20% by weight of such super absorbents, preferably from 5% to 15% by weight.

A mixture of super absorbents and hydrocolloids can be employed to render the acrylic adhesive hydrophilic. Suitable mixtures contain from 5% to 10% by weight of super absorbents and from 10% to 25% by weight of hydrocolloids.

Optionally, other materials may be included within the hydrophilic adhesive composition. For example, small amounts, i.e. less than 5% by weight, of a pharmaceutically active ingredient such as an antibiotic or antimicrobial agent, an antiseptic agent such as povidone iodine, a fragrance, an antioxidant.

The acrylic mass into which the water absorbing, water transmitting substances are added along with any optional materials can be any acrylic adhesive formulation known to be pressure sensitive and suitable for use on human skin. Suitable acrylics include acrylic esters particularly those with four or more carbon atoms in the alcohol component such as n-butyl acrylate and/or 2-ethylhexyl acrylate. The acrylic adhesive may contain other comonomers such as vinyl acetate, acrylonitrile, styrene, ethyl acrylate, methyl methacrylate, α,β-unsaturated carboxylic acids, esters, or half esters of unsaturated dicarboxylic acids. Terpolymers can also be used for this purpose. A discussion of pressure sensitive acrylic adhesives appears in the Handbook of Adhesives, 2nd Edition, Skeist, pages 543 - 552.

The hydrophilic, pressure sensitive, acrylic adhesive compositions are laminated to a polymeric foam layer and employed as a wound dressing. Suitable polymeric foams include semi-open or open cell polyurethane foams such as those employed by Chen in United States Patent US-A 3,972,328 and Pawelchak et al. in United States Patent US-A-4,538,603 and the flexible closed cell polyurethane foam containing one or more water dispersible, water swellable and/or water absorbing agents employed by Cilento et al in European Patent Application EP-A-190,814.

The hydrophilic, pressure sensitive acrylic adhesive compositions are prepared by blending and mixing the water/moisture absorbing, water/moisture transmitting substances and any optional substances, preferably in finely divided powder form, over a period of time into a slurry or suspension of the acrylic adhesive component in an organic solvent. Any conventional propeller blade type mixer can be employed and the blending is done at room temperature and normally takes from 15 to 30 minutes and results in an essentially homogeneous adhesive slurry. The resulting adhesive slurry is then cast onto a sheet of release paper or other suitable carrier at the desired thickness. The casting step is done with conventional apparatus such as a knife over roller to a thickness of from 40 to 100 mg. of adhesive per square inch depending upon the ultimate use. The adhesive coated release paper is then dried for example by passing through a hot air tunnel to evaporate off the organic solvent. The exposed surface of the adhesive can then be laminated to a polymeric foam as described above.

The adhesive compositions of this invention can be sterilized by means of gamma radiation.

The following examples are illustrative of the invention.

### Example 1

A wound dressing is prepared as follows.

An acrylic pressure sensitive adhesive slurry (40% solids in toluene/hexane, 30 kg.) commercially available from Avery as AS351® is placed within a propeller blade type mixer. A powdery mixture of sodium carboxymethylcellulose (3.84 kg.), pectin (0.48 kg.) and gelatin (0.48 kg.) is added with mixing over a period of 15 minutes and mixing is continued for an additional 5 minutes to give a homogeneous adhesive slurry.

This adhesive slurry is cast onto a sheet of silicone coated release paper by means of a knife over roller apparatus. The adhesive coated release paper is passed through a drying tunnel having a temperature of about 150°F (65.6°C) for about 15 minutes to evaporate off the toluene/hexane solvent to give an adhesive layer of about 75 mg. of dry adhesive per square inch.

The exposed surface of the adhesive is then pressure laminated to an open cell polyurethane foam layer. The resulting wound dressing is cut to shape, packaged, and sterilized.

### Examples 2-14

Following the procedures of Example 1, additional hydrophilic, pressure sensitive, acrylic adhesive compositions within the scope of this invention can be prepared for use in wound dressing products. The ingredients are expressed as the weight percent of the final dried adhesive mass:

| Ingredient | Weight percent of the ingredient within the dried adhesive composition | | | |
|---|---|---|---|---|
| | 2 | 3 | 4 | 5 |
| Acrylics | 70 | 65 | 70 | 90 |
| Sodium carboxymethylcellulose | 10 | 35 | 20 | - |
| Calcium carboxymethylcellulose | - | - | - | - |
| Pectin | 10 | - | - | - |
| Gelatin | 10 | - | - | - |
| Guar gum | - | - | - | - |
| Locust bean gum | - | - | - | - |
| Collagen | - | - | - | - |
| Gum karaya | - | - | - | - |
| Water insoluble starch-acrylonitrile graft copolymer(Water Lock A-100®) | - | - | 10 | 10 |
| Water insoluble cross-linked sodium carboxymethylcellulose | - | - | - | - |
| Water insoluble cross-linked dextran | - | - | - | - |

| Ingredient | Weight percent based upon total solids of the the adhesive formulation | | |
|---|---|---|---|
| | 6 | 7 | 8 |
| Acrylics | 85 | 65 | 70 |
| Sodium carboxymethylcellulose | - | - | - |
| Calcium carboxymethylcellulose | - | 20 | - |
| Pectin | - | - | 5 |
| Gelatin | - | - | 5 |
| Guar gum | - | - | 20 |
| Locust bean gum | - | 15 | - |
| Collagen | - | - | - |
| Gum karaya | - | - | - |
| Water insoluble starch-acrylonitrile graft copolymer(Water Lock A-100®) | - | - | - |
| Water insoluble cross-linked sodium carboxymethylcellulose | 15 | - | - |
| Water insoluble cross-linked dextran | - | - | - |

| Ingredient | Weight percent based upon total solids of the the adhesive formulation | | |
|---|---|---|---|
| | 9 | 10 | 11 |
| Acrylics | 70 | 70 | 75 |
| Sodium carboxymethylcellulose | 20 | 20 | 20 |
| Calcium carboxymethylcellulose | - | - | - |
| Pectin | - | - | - |
| Gelatin | 5 | - | - |
| Guar gum | - | - | - |
| Locust bean gum | 5 | - | - |
| Collagen | - | - | - |
| Gum karaya | - | 10 | - |
| Water insoluble starch-acrylonitrile graft copolymer(Water Lock A-100®) | - | - | - |
| Water insoluble cross-linked sodium carboxymethylcellulose | - | - | - |
| Water insoluble cross-linked dextran | - | - | - |

| Ingredient | Weight percent based upon total solids of the the adhesive formulation | | |
|---|---|---|---|
| | 12 | 13 | 14 |
| Acrylics | 80 | 55 | 88 |
| Sodium carboxymethylcellulose | 15 | 25 | - |
| Calcium carboxymethylcellulose | - | - | - |
| Pectin | - | 10 | - |
| Gelatin | - | 10 | - |
| Guar gum | - | - | - |
| Locust bean gum | - | - | - |
| Collagen | - | - | - |
| Gum karaya | - | - | - |
| Water insoluble starch-acrylonitrile graft copolymer(Water Lock A-100®) | 5 | - | - |
| Water insoluble cross-linked sodium carboxymethylcellulose | - | - | - |
| Water insoluble cross-linked dextran | - | - | 12 |

## Claims

1. A process for preparing a wound dressing comprising a hydrophilic adhesive layer suitable for use on the human skin comprising a pressure sensitive acrylic adhesive mass containing one or more water/moisture absorbing, water/moisture transmitting substances, wherein said water/moisture absorbing, water/moisture transmitting substances are (i) one or more hydrocolloids selected from sodium carboxymethylcellulose, calcium carboxymethylcellulose, pectin, gelatin, guar gum, locust bean gum, collagen, and gum karaya, (ii) one or more super absorbents selected from substantially water insoluble starch-acrylonitrile graft copolymers, substantially water insoluble cross-linked sodium carboxymethylcellulose, and substantially water insoluble cross-linked dextran, or (iii) mixtures of one or more of said hydrocolloids and super absorbents, said process comprising:
a) adding a solution of the pressure sensitive acrylic adhesive in an organic solvent to a propeller type mixer,
b) blending into said acrylic solution a powder comprising the one or more water/moisture absorbing, water/moisture transmitting substances,
c) continuing said blending until a homogeneous slurry is formed,
d) casting the resulting slurry of hydrophilic, pressure sensitive, adhesive onto a sheet of release carrier, to a thickness which provides from 40 to 100 mg of hydrophilic, pressure sensitive, adhesive per square inch (6.452 x 10⁻⁴ m²) after drying,
e) drying said adhesive slurry to evaporate off said organic solvent, and
f) laminating the resulting hydrophilic adhesive layer to a polymeric foam layer.

2. The process of claim 1 wherein said water/moisture absorbing, water/moisture transmitting substances are one or more hydrocolloids present in the hydrophilic acrylic adhesive composition at from 15% to 50% by weight of said acrylic adhesive mass.

3. The process of claim 2 wherein said hydrocolloids are a powdery blend of sodium carboxymethylcellulose, pectin, and gelatin and said blend is present in the hydrophilic acrylic adhesive at from 20% to 40% by weight of said acrylic adhesive mass.

4. The process of claim 1 wherein said water/moisture absorbing, water/moisture transmitting substances are one or more super absorbents present in the hydrophilic acrylic adhesive composition at from 5% to 20% by weight of said acrylic adhesive mass.

5. The process of claim 4 wherein said super absorbent is a water insoluble starch-acrylonitrile graft copolymer present in the hydrophilic adhesive composition at from 5% to 15% by weight of said acrylic adhesive mass.

6. The process of claim 1 wherein said water/moisture absorbing, water/moisture transmitting substances are a mixture from 5% to 10% by weight of said acrylic adhesive mass of one or more super absorbents and from 10% to 25% by weight of said acrylic adhesive mass of one or more hydrocolloids.

## Patentansprüche

1. Verfahren zur Herstellung eines Pflasters, das eine zur Verwendung auf der menschlichen Haut geeignete hydrophile Haftschicht umfaßt, die eine ein oder mehrere Wasser/Feuchtigkeit absorbierende, Wasser/Feuchtigkeit übertragende Stoffe enthaltende druckempfindliche Acrylat-Klebstoffmasse umfaßt, wobei die Wasser/Feuchtigkeit absorbierenden, Wasser/Feuchtigkeit übertragenden Stoffe (i) ein oder mehrere Hydrokolloide, ausgewählt aus Natriumcarboxymethylcellulose, Calciumcarboxymethylcellulose, Pektin, Gelatine, Guargummi, Johannisbrotgummi, Kollagen und Karayagummi, (ii) ein oder mehrere Superabsorptionsmittel, ausgewählt aus im wesentlichen wasserunlöslichen Stärke-Acrylnitril-Pfropfcopolymeren, im wesentlichen wasserunlöslicher vernetzter Natriumcarboxymethylcellulose und im wesentlichen wasserunlöslichem vernetztem Dextran, oder (iii) Gemische aus einem oder mehreren dieser Hydrokolloide und Superabsorptionsmitteln sind, das Verfahren umfassend:
a) die Zugabe einer Lösung des druckempfindlichen Acrylatklebstoffs in einem organischen Lösungsmittel zu einem Propellermischer-Typ,
b) Mischen eines Pulvers, das ein oder mehrere Wasser/Feuchtigkeit absorbierende, Wasser/Feuchtigkeit über tragende Stoffe umfaßt, in die Acrylatlösung,
c) Fortsetzen des Mischens bis eine homogene Aufschlämmung erzeugt wird,
d) Gießen der erhaltenen Aufschlämmung des hydrophilen druckempfindlichen Klebstoffs auf einen Bogen Ablöseträger zu einer Dicke, die nach dem Trocknen 40 bis 100 mg des hydrophilen, druckempfindlichen Klebstoffs pro Quadratszoll (6,452 x 10⁻⁴ m²) bereitstellt,
e) Trocknen der Klebstoffaufschlämmung, wobei das organische Lösungsmittel verdampft und
f) Laminieren der erhaltenen hydrophilen Klebstoffschicht auf eine polymere Schaumschicht.

2. Verfahren nach Anspruch 1, wobei die Wasser/Feuchtigkeit absorbierenden, Wasser/Feuchtigkeit übertragenden Stoffe ein oder mehrere Hydrokolloide sind, die in der hydrophilen Acrylatklebstoffzusammensetzung in einer Menge von 15 bis 50 Gew.-% der Acrylatklebstoffmasse vorliegen.

3. Verfahren nach Anspruch 2, wobei die Hydrokolloide eine pulverförmige Mischung von Natriumcarboxymethylcellulose, Pektin und Gelatine sind und die Mischung in dem hydrophilen Acrylatklebstoff in einer Menge von 20 bis 40 Gew.-% der Acrylatklebstoffmasse vorliegt.

4. Verfahren nach Anspruch 1, wobei die Wasser/Feuchtigkeit absorbierenden, Wasser/Feuchtigkeit übertragenden Stoffe ein oder mehrere Superabsorptionsmittel sind, die in der hydrophilen Acrylatklebstoffzusammensetzung in einer Menge von 5 bis 20 Gew.-% der Acrylatklebstoffmasse vorliegen.

5. Verfahren nach Anspruch 4, wobei das Superabsorptionsmittel ein in Wasser unlösliches Stärke-Acrylnitril-Pfropfcopolymer ist, das in der hydrophilen Klebstoffzusammensetzung in einer Menge von 5 bis 15 Gew.-% der Acrylatklebstoffmasse vorliegt.

6. Verfahren nach Anspruch 1, wobei die Wasser/Feuchtigkeit absorbierenden, Wasser/Feuchtigkeit übertragenden Stoffe ein Gemisch aus einem oder mehreren Superabsorptionsmitteln entsprechend 5 bis 10 Gew.-% der Acrylatklebstoffmasse und aus ein oder mehreren Hydrokolloiden entsprechend 10 bis 25 Gew.-% der Acrylatklebstoffmasse sind.

## Revendications

1. Procédé de préparation d'un pansement pour plaies comprenant une couche d'adhésif hydrophile convenant à l'utilisation sur la peau humaine, comprenant une masse d'adhésif acrylique sensible à la pression renfermant une ou plusieurs substances qui absorbent l'eau et l'humidité et qui transmettent l'eau et l'humidité, lesdites substances qui absorbent l'eau et l'humidité et qui transmettent l'eau et l'humidité étant (i) un ou plusieurs hydrocolloïdes choisis entre la carboxyméthylcellulose sodique, la carboxyméthylcellulose calcique, la pectine, la gélatine, le guar, la gomme tragasol, le collagène, et le karaya, (ii) un ou plusieurs super absorbants choisis entre les copolymères greffés d'amidon et d'acrylonitrile, pratiquement insolubles dans l'eau, la carboxyméthylcellulose sodique réticulée, pratiquement insoluble dans l'eau, et le dextranne réticulé pratiquement insoluble dans l'eau, ou (iii) des mélanges d'un ou plusieurs desdits hydrocolloïdes et super absorbants, ledit procédé consistant:
a) à placer une solution de l'adhésif acrylique sensible à la pression dans un solvant organique, dans un mélangeur à hélice,
b) à mélanger à ladite solution d'adhésif acrylique une poudre comprenant la ou les substances qui absorbent l'eau et l'humidité et qui transmettent l'eau et l'humidité,
c) à continuer ledit mélange jusqu'à ce qu'il se forme une dispersion homogène,
d) à couler la dispersion résultante d'adhésif hydrophile et sensible à la pression sur une feuille de support anti-adhésif, sur une épaisseur correspondant à 40 à 100 mg d'adhésif hydrophile et sensible à la pression par pouce carré (6,452 x 10⁻⁴ m²) après séchage,
e) à faire sécher ladite dispersion d'adhésif pour faire évaporer ledit solvant organique, et
f) à stratifier la couche d'adhésif hydrophile résultante sur une couche de mousse polymère.

2. Procédé selon la revendication 1, dans lequel lesdites substances qui absorbent l'eau et l'humidité et qui transmettent l'eau et l'humidité sont un ou plusieurs hydrocolloïdes présents dans la composition d'adhésif acrylique hydrophile à raison de 15% à 50% du poids de ladite masse d'adhésif acrylique.

3. Procédé selon la revendication 2, dans lequel lesdits hydrocolloïdes sont un mélange poudreux de carboxyméthylcellulose sodique, de pectine et de gélatine, et ledit mélange est présent dans l'adhésif acrylique hydrophile à raison de 20% à 40% du poids de ladite masse d'adhésif acrylique.

4. Procédé selon la revendication 1, dans lequel lesdites substances qui absorbent l'eau et l'humidité et qui transmettent l'eau et l'humidité sont un ou plusieurs super absorbants présents dans la composition d'adhésif acrylique hydrophile à raison de 5% à 20% du poids de ladite masse d'adhésif acrylique.

5. Procédé selon la revendication 4, dans lequel ledit super absorbant est un copolymère greffé d'amidon et d'acrylonitrile insoluble dans l'eau, présent dans la composition d'adhésif hydrophile à raison de 5% à 15% du poids de ladite masse d'adhésif acrylique.

6. Procédé selon la revendication 1, dans lequel lesdites substances qui absorbent l'eau et l'humidité et qui transmettent l'eau et l'humidité sont un mélange de 5% à 10%, en poids de ladite masse d'adhésif acrylique, d'un ou plusieurs super absorbants, et de 10% à 25%_{,} en poids de ladite masse d'adhésif acrylique, d'un ou plusieurs hydrocolloïdes.
